Europäisches Patentamt

European Patent Office

Office européen des brevets

(19)

(11) Publication number: 0 523 165 B1

(12) EUROPEAN PATENT SPECIFICATION

(45) Date of publication of patent specification: 29.11.95    (51) Int. Cl.$^6$: C07C 2/68

(21) Application number: 91907921.0

(22) Date of filing: 04.04.91

(86) International application number:
PCT/US91/02337

(87) International publication number:
WO 91/15443 (17.10.91 91/24)

(54) NAPHTHALENE ALKYLATION PROCESS.

(30) Priority: 06.04.90 US 505392
22.10.90 US 600887

(43) Date of publication of application:
20.01.93 Bulletin 93/03

(45) Publication of the grant of the patent:
29.11.95 Bulletin 95/48

(84) Designated Contracting States:
BE DE FR GB IT NL

(56) References cited:
EP-A- 0 160 144
US-A- 3 716 596
US-A- 4 871 444
US-A- 4 876 408
US-A- 4 912 277

(73) Proprietor: MOBIL OIL CORPORATION
3225 Gallows Road
Fairfax,
Virginia 22037-0001 (US)

(72) Inventor: ASHJIAN, Henry
22 Surrey Lane
East Brunswick, NJ 08033 (US)
Inventor: LE, Ouang, Ngoc
330 Rhode Island Avenue
Cherry Hill, NJ 08002 (US)
Inventor: LISSY, Daria, Nowakiwska
404 South Ivy Lane
Glen Mills, PA 19342 (US)
Inventor: MARLER, David, Owen
801 Cooper Street 272B
Deptford, NJ 08096 (US)
Inventor: SHIM, Joosup
6 South Marion Avenue
Wenonah, NJ 08090 (US)
Inventor: WONG, Stephen, Sui, Fai Jurong Re-
finery
Mobil Oil Singapore P.T.E. Ltd.
Singapore 9059 (SG)

Rank Xerox (UK) Business Services
(3.10/3.09/3.3.3)

(74) Representative: **Colmer, Stephen Gary**
**European Office of Patent Counsel**
**Mobil House**
**500-600 Witan Gate**
**Central Milton Keynes**
**Buckinghamshire MK9 1ES (GB)**

**Description**

This invention relates to the production of alkylated naphthalenes and substituted naphthalenes.

Alkylaromatic fluids have been proposed for use as certain types of functional fluids where good thermal and oxidative are required. For example, U.S. Patent No. 4,714,794 (Yoshida) describes the monoalkylated naphthalenes as having excellent thermal and oxidative stability, low vapor pressure and flash point, good fluidity and high heat transfer capacity and other properties which render them suitable for use as thermal medium oils. The use of a mixture of monoalkylated and polyalkylated naphthalenes as a base for synthetic functional fluids is described in U.S. Patent no. 4,604,491 (Dressler) and Pellegrini U.S. 4,211,665 and 4,238,343 describe the use of alkylaromatics as transformer oils.

The alkylated naphthalenes are usually produced by the alkylation of naphthalene or a substituted naphthalene in the presence of an acidic alkylation catalyst such as a Friedel-Krafts catalyst, for example, an acidic clay as described in Yoshida U.S. 4,714,794 or Dressler U.S. 4,604,491 or a Lewis acid such as aluminum trichloride as described in Pellegrini U.S. 4,211,665 and 4,238,343. The use of a catalyst described as a collapsed silica-alumina zeolite as the catalyst for the alkylation of aromatics such as naphthalene is disclosed in Boucher U.S. 4,570,027. The use of various zeolites including intermediate pore size zeolites such as ZSM-5 and large pore size zeolites such as zeolite L and ZSM-4 for the alkylation of various monocyclic aromatics such as benzene is disclosed in Young U.S. 4,301,316 and Bowes US 3,716,596

In the formulation of functional fluids based on the alkyl naphthalenes, it has been found that the preferred alkyl naphthalenes are the mono-substituted naphthalene since they provide the best combination of properties in the finished product: because the mono-alkylated naphthalenes posses fewer benzylic hydrogens than the corresponding di-substituted or polysubstituted versions, they have better oxidative stability and therefore form better functional fluids and additives. In addition, the mono-substituted naphthalenes have a kinematic viscosity in the desirable range of about 5-8 cSt (at 100°C.) when working with alkyl substituents of about 14 to 18 carbon atoms chain length. Although the mono-alkylated naphthalenes may be obtained in admixture with more highly alkylated naphthalenes using conventional Friedel-Krafts catalysts such as those mentioned above or by the use of zeolites such as USY, the selectivity to the desired mono-alkylated naphthalenes is not obtained.

We have now found that zeolite catalysts can be effective for the production of mono-alkylated naphthalenes and that good selectivity for the mono-substituted naphthalenes may be obtained by the incorporation of cations having a radius of at least 2.5 Å in large pore size zeolites such as zeolite Y. The presence of bulky cations of this type provides good selectivity for the more highly desired mono-alkylated products.

The present invention provides a process for preparing long chain alkyl substituted naphthalenes which comprises reacting a naphthalene with an alkylating agent possessing an alkylating aliphatic group having at least six carbon atoms under alkylation reaction conditions and in the presence of an alkylation catalyst comprising a porous crystalline zeolite containing cations having a radius of at least 2.50 A. to form an alkylated naphthalene possessing at least one alkyl group derived from the alkylating agent.

The starting materials for the production of the alkylated naphthalenes are naphthalene itself as well the substituted naphthalenes which may contain one or more short chain alkyl groups containing up to about eight carbon atoms, such as methyl, ethyl or propyl. Suitable alkyl-substituted naphthalenes include alpha-methylnaphthalene, dimethylnaphthalene and ethylnaphthalene. Naphthalene itself is preferred since the resulting mono-alkylated products have better thermal and oxidative stability than the more highly alkylated materials for the reasons set out above.

The alkylating agents which are used to alkylate the naphthalene include any aliphatic or aromatic organic compound having one or more available alkylating aliphatic groups capable of alkylating the naphthalene . The alkylatable group itself should have at least 6 carbon atoms, preferably at least 8, and still more preferably at least 12 carbon atoms. For the production of functional fluids and additives, the alkyl groups on the alkyl-naphthalene preferably have from 12 to 30 carbon atoms, with particular preference to 14 to 18 carbon atoms. A preferred class of alkylating agents are the olefins with the requisite number of carbon atoms, for example, the hexenes, heptenes, octenes, nonenes, decenes, undecenes, dodecenes. Mixtures of the olefins, e.g. mixtures of $C_{12}$-$C_{20}$ or $C_{14}$-$C_{18}$ olefins, are useful. Branched alkylating agents, especially oligomerized olefins such as the trimers, tetramers, pentamers, etc., of light olefins such as ethylene, propylene, the butylenes, etc., are also useful. Other useful alkylating agents which may be used, although less easily, include alcohols (inclusive of monoalcohols, dialcohols, trialcohols, etc.) such as hexanols, heptanols, octanols, nonanols, decanols, undecanols and dodecanols; and alkyl halides such as hexyl chlorides, octyl chlorides, dodecyl chlorides; and higher homologs.

The alkylation reaction between the naphthalene and the alkylating agent is carried out in the presence of a zeolite catalyst which contains a cation of certain specified radius. The molecular size of the alkylation products will require a relatively large pore size in the zeolite in order for the products to leave the zeolite, indicating the need for a relatively large pore size in the zeolite, which will also tend to reduce diffusion limitations with the long chain alkylating agents. The large pore size zeolites are the most useful zeolite catalysts for this purpose although the less highly constrained intermediate pore size zeolites may also be used, as discussed below. The large pore size zeolites are zeolites such as faujasite, the synthetic faujasites (zeolites X and Y), zeolite L, ZSM-4, ZSM-18, ZSM-20, mordenite and offretite which are generally useful for this purpose. These large pore zeolites are characterised by the presence of a 12-membered oxygen ring system in the molecular structure and by the existence of pores with a minimum dimension of at least 7.4 Å, as described by Frilette et al. in J. Catalysis 67,218-222 (1981). See also Chen et al. Shane-Selective Catalysis in Industrial Applications, (Chemical industries; Vol. 36) Marcel Dekker Inc., New York 1989, ISBN 0-8247-7856-1 and Hoelderich et al. Angew. Chem. Int. Ed. Engl. 27 226-246 (1988), especially pp.226-229. The large pore size zeolites may also be characterised by a "Constraint Index" of not more than 2, in most cases not more than 1. Zeolite beta, a zeolite having a structure characterised by twelve-membered pore openings, is included in this class of zeolites although under certain circumstances it has a Constraint Index approaching the upper limit of 2 which is usually characteristic of this class of zeolites. The method for determining Constraint Index is described in U. S. Patent No. 4,016,218, together with values for typical zeolites and of the significance of the Index in U.S. Patent No.4,861,932, to which reference is made for a description of the test procedure and its interpretation.

Zeolites whose structure is that of a ten membered oxygen ring, generally regarded as the intermediate pore size zeolites may also be effective catalysts for this alkylation reaction if their structure is not too highly constrained. Thus, zeolites such as ZSM-12 (Constraint Index 2) may be effective catalysts for this reaction. The zeolite identified as MCM-22 is a useful catalyst for this reaction. MCM-22 is described in U.S. Patent Application Serial No. 07/254524, filed 6 October 1988 and also in International Patent Application PCT/US 88/04251, to which reference is made for a description of this zeolite. Thus, zeolites having a Constraint Index up to about 3 will generally be found to be useful catalysts, although the activity may be found to be dependent on the choice of alkylating agent, especially its chain length, a factor which imposes diffusion limitations upon the choice of zeolite.

A highly useful zeolite for the production of the mono-alkylated naphthalenes is zeolite Y in the ultrastable form, usually referred to as USY. When this material contains hydrated cations, it catalyses the alkylation in good yields with excellent selectivity. Zeolite USY is a material of commerce, available in large quantities as a catalyst for the cracking of petroleum. It is produced by the stabilisation of zeolite Y by a procedure of repeated ammonium exchange and controlled steaming. Processes for the production of zeolite USY are described in U. S. Patents Nos. 3,402,966 (McDaniel), 3,923,192 (Maher) and 3,449,070 (McDaniel); see also Wojciechowski, Catalytic Cracking, Catalysts, Chemistry and Kinetics, (Chemical Industries Vol. 25), Marcel Dekker, New York, 1986, ISBN 0-8247-7503-8, to which reference is made for a description of zeolite USY, its preparation and properties.

The selected zeolite catalyst contains a cation which has a radius of at least 2.5 Å, and preferably at least 3.0 Å. A number of cations conform to this requirement, including the hydrated cations of a number of metals, including monovalent, divalent and polyvalent, transitional and non-transitional metals. Even though the non-hydrated cations may not themselves conform to the ionic size requirement, the hydrated forms of the cations may do so. In particular, the relatively small radius cations of the alkali metals such as sodium and lithium (ionic radii of 0.95 and 0.60 Å, respectively) do not conform to the requirement, but the hydrated forms of these cations readily meet the requirement (radii of 3.58 and 3.82 Å). In this respect, it is noteworthy that the more intense electric fields surrounding the smaller size non-hydrated cations produce a more intense polarisation of the water molecules so that the radii of the hydrated cations are greater; this effect is readily observed in the case of the sodium and lithium cations, whose hydrated and non-hydrated radii are noted above. A variety of cationic forms of the zeolite may therefore be used. Typical cations which may be used include the hydrated cations of metals of Group IA of the Periodic Table (IUPAC Table), especially sodium or potassium, divalent cations, especially of Group IIA e.g. calcium, and cations of the Rare Earths e.g. cerium, yttrium, lanthanum. The hydrated ammonium cation is also a suitable cationic form of the zeolite and is often preferred for zeolite Y or USY since these zeolites may be commercially available in the ammonium form as a precursor of the decationised or hydrogen form of the zeolite. The hydrated protonic form of the zeolite i.e. where the cation is the hydronium ion $H_3O$, is also effective as a catalyst.

Cations of the required radius may also be provided by various organic species, especially the organic nitrogenous bases. A preferred class of cations of this type are the substituted ammonium cations, for example, alkylammonium cations, especially the short chain alkylammonium cations e.g. tetramethylammonium (TMA), tetraethylammonium (TEA) or tetrapropylammonium (TPA). Short chain alkyl groups up to $C_6$ are generally useful for cations of this kind.

The ionic radii of selected cations in the hydrated and non-hydrated forms are given below in Table 1.

Table 1

| Cationic Radii (Å) | | |
|---|---|---|
| Monovalent | Ionic Radius (Å) | Hydrated Radius (Å) |
| H | <0.50 | - |
| $H_3O$ | 1.50 | 2.82 |
| Cs | 1.69 | 3.29 |
| $NH_4$ | 1.48 | 3.31 |
| K | 1.33 | 3.31 |
| Ag | 1.26 | 3.41 |
| Na | 0.95 | 3.58 |
| Li | 0.60 | 3.82 |
| TMA | 3.20 | 3.20 |
| TEA | 3.95 | 3.95 |
| TPA | 4.50 | 4.50 |
| Divalent | | |
| Ca | 0.99 | 4.2 |
| Mg | 0.65 | 4.4 |
| Cu | 0.72 | 4.2 |
| Zn | 0.74 | 4.3 |
| Ni | 0.70 | 4.04 |
| $Pt(NH_3)_4$ | - | >3 |
| Trivalent | | |
| La | 1.15 | 4.52 |

From the Table above it can be seen that hydrated cations will generally conform to the ionic size requirement, even if the non-hydrated form of the cation does not. Thus, for example, the non-hydrated forms of the alkali metal and hydronium cations are too small for the present purposes but when hydrated, the ionic radii increase to a value which is sufficient to achieve the desired increases in alkylation selectivity and activity.

If the zeolite is not already in the desired ionic form, the cation may be introduced by ion-exchange in the conventional manner using a solution of the exchanging cation. The use of an aqueous solution for the ion exchange results in the ion being in the hydrated form after the exchange is complete. Non-hydrated cations may be converted to the hydrated form by exposing the zeolite to hydrating conditions.

Since the improvements in catalytic function are related to the presence of the cations of the required ionic size in the zeolite catalyst, the cations should be present in the zeolite in a sufficient amount to achieve the desired effect. It is not essential for the cation exchange to be complete for the improvements to be obtained and generally, a single cation exchange procedure should be sufficient to achieve the desired improvements.

The presence of the bulky cations in the zeolite is thought to selectively block off the interior channels in the zeolite so that production of more highly alkylated species above the mono-alkylated naphthalenes is effectively prevented, either by product size exclusion or spatiospecificity, as described by Chen et al. In any event, the selectivity of the large pore zeolite catalyst is altered notably towards production of the mono-alkylated naphthalene , as compared to the characteristic mixture of various alkylated species obtained with the unmodified zeolite in the hydrogen form. The zeolite may be composited with a matrix material or binder which is resistant to the temperatures and other conditions employed in the alkylation process. Such materials include active and inactive materials and synthetic or naturally occurring zeolites as well as inorganic materials such as clays, silica and/or metal oxides such as alumina, silica or silica-alumina. The latter may be either naturally occurring or in the form of gelatinous precipitates or gels including mixtures of silica and metal oxides. Use of an active material in conjunction with the zeolite may change the conversion and/or selectivity of the catalyst. Inactive materials suitably serve as diluents to control the amount of conversion so that alkylation products can be obtained economically and orderly without employing other means for controlling the rate of reaction. Binders which may be incorporated to improve the crush strength and other physically properties of the catalyst under commercial alkylation operating conditions include naturally occurring clays, e.g., bentonite and kaolin as well as the oxides referrd to above.

The relative proportions of zeolite, present in finely divided crystalline form oxide matrix may vary widely, with the crystalline zeolite content ranging from about 1 to about 90 percent by weight and more usually, particularly when the composite is prepared in the form of beads, in the range of about 2 to about 80 weight percent of the composite.

The stability of the alkylation catalyst of the invention may be increased by steaming. U.S. Patent Nos. 4,663,492; 4,594,146; 4,522,929; and 4,429,176, describe conditions for the steam stabilization of zeolite catalysts which can be utilized to steam-stabilize the catalyst. The steam stabilization conditions include contacting the catalyst with, e.g., 5-100% steam at a temperature of at least about 300°C (e.g., 300-650°C) for at least one hour (e.g., 1-200 hours) at a pressure of 100 - 2,500 kPa, e.g. steaming with 75-100% steam at 315°-500°C and atmospheric pressure for 2-25 hours. The steam stabilization treatment may, as described in the above-mentioned patents, take place under conditions sufficient to initially increase the Alpha Value of the catalyst, and produce a steamed catalyst having a peak Alpha Value. If desired, steaming can be continued to subsequently reduce the Alpha Value from the peak Alpha Value to an Alpha Value which is substantially the same as the Alpha Value of the unsteamed catalyst.

In a further embodiment of this invention, the aromatic nucleus is alkylated with alkylating agents which contain at least one long chain constituent particularly an alkylating agent in which the alkylating constituent is a long chain alpha olefin, in effective contact with heterogeneous, acidic, relatively large pore alkylation catalysts of limited acid activity, that is, lower acid activity than was previously used to catalyze these reactions.

It has been found that, when this alkylation reaction is carried out using such catalysts with such limited acid activity, the conversion achieved during the reaction is markedly increased, even when operated under the same reaction conditions. This is most surprising since, if alkylation is an acid catalyzed reaction, one would expect that higher acid activity would improve the conversion, and that lower acid activity would reduce conversion, exactly the opposite of what has been found and upon what this invention is based. Additionally, it has been unexpectedly found that, not only is the conversion efficiency of this reaction improved, but the yield of products suited to use in lubricant compositions is also increased, and the properties of this improved product yield are improved as well.

Another and preferred aspect of this embodiment of this invention lies in post formation reducing the acid activity of the catalyst comprising the zeolite to a desired lower level after the zeolite has been initially made at a higher acid activity. Thus, it has been found to be desirable to produce a large pore zeolite having substantial acid activity, and then to reduce its acid activity by a post treatment procedure such as steaming or cation exchange.

One of the known methods of measuring the acid activity of a catalyst is to determine its alpha value. Reference is here made to Journal of Catalysis, Vol. 4, p. 527 (1965); vol. 6, p.278 (1966); and vol. 61, p.278 (1980), for a full description of the techniques for carrying out the Alpha test. It should be noted in passing, that the alpha value of a highly active amorphous silica-alumina catalyst has been arbitrarily taken as 1, and all other acid activities compared to that. In accord with this embodiment of the invention, catalysts which have an alpha value of 0.5 to 60 are useful in this improved process. It is preferred for the catalyst alpha to be 1 to 60, most preferred are those alkylation catalysts which have been formulated from materials such that they have a substantially higher acid activity and are then treated, after catalyst formation, to reduce their acid activity to the desired level of below about 60 alpha.

It is a preferred form of this invention, to modify the crystal composition of the zeolite crystal after it is made, by post steam treatment, cation exchange or framework isomorphous substitution for example, in order to achieve a material having the desired acid activity. Techniques for initially manufacturing zeolite crystals as well as for modifying such crystals after they have been made are well known and have been widely published.

It is well known that the acid activity of zeolite crystals can be modified by post formation treatment with steam under certain conditions. This invention takes advantage of such known steam treatment to produce a catalyst with controlled acid activity for use in an improved aromatics alkylation process. No invention is here claimed in the steam treatment per se.

Thus the zeolite crystal which is to be used as a catalyst for this process, can be treated with steam at a temperature of at least 300 °C, preferably 300 to 650 °C, for at least one (1) hour, for example from 1 to 200 hours, at a pressure of from 100 to 2,500 kPa. More specifically, a steaming treatment which has been found to be well suited to use in this invention is to subject either the zeolite crystal or the fully formulated catalyst, including crystal and binder, to an atmosphere of from 75 to 100 % steam at 315 to 500 °C at atmospheric pressure for 2 to 25 hours. Thus, zeolite crystals of high acid activity (high alpha) can initially be made and these materials then steamed to reduce their acid activity, as measured by the alpha test, to an acceptable level.

It is also within the scope of this invention to modify the acid activity of the catalytic material, comprising zeolite crystals, by cation exchanging a predetermined number of the acid sites of the crystals with non-catalytic cations such as those of Groups IA and IIA of the Periodic Table, preferably sodium. When this technique is used, it is often desirable to reexchange the acid sites periodically during the alkylation process of this invention, preferably as part of the regeneration procedure. It may also be appropriate to control the acid activity of the catalytic solid by preapplying a predetermined proportion of coke thereon, or by allowing a certain predeterminer proportion of coke to build up on the catalyst before taking product from the process. The particular means of controlling the acid activity is not critical to the practice of this invention, so long as the alpha activity of the catalytic solid is maintained as aforesaid. However, steaming is preferred.

The single figure of the drawing is a curve which shows the relationship between conversion and acid activity for a catalyst based on a zeolite crystalline solid having the topology of zeolite Beta and consisting predominantly of silicon, aluminum and oxygen.

The alkylation process of this invention is conducted such that the organic reactants, i.e., the alkylatable aromatic compound and the alkylating agent, are brought into contact with the zeolite catalyst in a suitable reaction zone such as, for example, in a flow reactor containing a fixed bed of the catalyst composition, under effective alkylation conditions. Such conditions typically include a temperature of from 100°C to 400°C, a pressure of from 20 to 25000 kPa (0.2 to 250 atmospheres), a feed weight hourly space velocity (WHSV) of from 0.1 $hr^{-1}$ to 10 $hr^{-1}$ and an alkylatable aromatic compound to alkylating agent mole ratio of from 0.1:1 to 50:1, preferably from 4:1 to 1:4 e.g. from 2:1 to 1:2. The WHSV is based upon the weight of the catalyst composition employed, i.e., the total weight of active catalyst (and binder if present). Preferred reaction conditions include a temperature within the approximate range of from 100°C to 350°C, a pressure of from 100 to 2500 kPa (1 to 25 atmospheres), a WHSV of from 0.5 $hr^{-1}$ to 5 $hr^{-1}$ and an alkylatable aromatic compound to alkylating agent mole ratio of from 0.5:1 to 5:1. When using naphthalene as the aromatic compound, the pressure should preferably be maintained at a value of at least about 50 psig in order to prevent the naphthalene from subliming into the overhead of the alkylation reactor; the required pressure may be maintained by inert gas pressurization, preferably with nitrogen. The reactants can be in either the vapor phase or the liquid phase and can be neat, i.e., free from intentional admixture or dilution with other material, or they can be brought into contact with the zeolite catalyst composition with the aid of carrier gases or diluents such as, for example, hydrogen or nitrogen. The alkylation can be carried out as a batch-type reaction typically employing a closed, pressurized, stirred reactor with an inert gas blanketing system or in a semi-continuous or continuous operation utilizing a fixed or moving bed catalyst system.

The products comprising alkylated aromatics are characterized by exceptional oxidative and thermal stability. They may be separated from the reaction mixture by stripping off unreacted alkylating agent and naphthalene compound in the conventional manner. It has also been found that the stability of the alkylated product may be improved by filtration over activated charcoal and by alkali treatment to remove impurities, especially acidic by-products formed by oxidation during the course of the reaction. The alkali treatment is preferably carried out by filtration over a solid alkali material, preferably calcium carbonate (lime). In a typical product work-up, it has been found, for example, that the RBOT (Rotating Bomb Oxidation Test) stability can be increased from a value of 184 minutes for an unstripped product ($C_{14}$-alkylnaphthalene) to 290 minutes if the unreacted materials are removed by stripping and to 350 minutes if the stripped product is filtered over lime ($CaCO_3$).

EXAMPLES

Example 1

This Example demonstrates the catalytic activity of a conventional, calcined USY zeolite in $H^+$ form for alkylating naphthalene with a long chain alpha olefin to produce alkylated naphthalene lube base stocks. The catalyst used in this example is an unbound USY catalyst (100% zeolite) containing only about 0.47 wt. percent sodium and having an unit cell size of 24.51 Å (Catalyst A). The alkylation experiment was carried out in a one liter autoclave using 2:1 molar ratio of alpha-$C_{14}$ = :naphthalene, 2 wt percent catalyst at 204°C (400°F) for 6 hours under a nitrogen pressure of 1 atmosphere. After decanting and filtering the catalyst, the total liquid product was then vacuum distilled at 316°C (600°F) to obtain 26 wt percent alkylated lube base stock comprising 85% mono-, 8% di-alkylated naphthalenes, and in addition, 7% olefin dimer due to olefin oligomerization. This corresponds to the conversion of 38 wt percent naphthalene and 22 wt percent alpha $C_{14}$ olefin.

Example 2

In this Example, the alkylation reaction was carried out under identical conditions as in Example 1 except the USY catalyst loading was increased from 2 to 5 wt percent. As expected, the conversion increases with a higher zeolite loading. The production of alkylated naphthalene lube increases from 26 wt percent (Example 1) to 54 wt percent when the zeolite loading increases from 2 to 5 w percent. Table 2 compares the product selectivity and catalyst activity as a function of zeolite loading.

Table 2

| Example No | 1 | 2 |
|---|---|---|
| USY Zeolite, wt. pct. | 2 | 5 |
| Conversion, wt. pct.: | | |
| Naphthalene | 38 | 83 |
| Alpha $C_{14}$ Olefin | 22 | 45 |
| Total Alkylated Lube, wt. pct. | 26 | 54 |
| Product Distribution,wt. pct.: | | |
| Mono-Alkylated | 85 | 80 |
| Di-Alkylated | 8 | 17 |
| Dimer | 7 | 3 |

As the conversion of reactants increases, the product selectivity shifts from mono- to di-alkylated products from 8 percent (Example 1) to 17 percent (Example 2).

Example 3

This Example illustrates the catalytic performance of USY zeolite in the hydrated ammonium form. The experiment was carried under identical process conditions as in Example 1 but using a hydrated ammonium USY zeolite instead of the hydrogen form USY (Catalyst A). The hydrated ammonium USY zeolite was obtained from the $H^+$ USY by ion-exchanging with a solution of ammonium nitrate as follows: $H^+$ USY zeolite (Catalyst A) was slurried with 0.5 N $(NH_4)_2SO_4$, 5 ml of solution per gram of zeolite. After 1 hour stirring was stopped and solids were allowed to settle. The liquid was then decanted and the exchange procedure repeated. After the second exchange, the zeolite was washed with deionized water until sulfate-free and then dried at 121°C (250°F). The resulting hydrated ammonium catalyst contains 0.32 wt. percent sodium and 0.47 wt. percent nitrogen (Catalyst B). Table 3 compares the alkylation performance of USY catalyst in the hydrated ammonium form with the unhydrated hydrogen form.

Table 3

| Example No | 1 | 3 |
|---|---|---|
| Catalyst<br>USY Form | A<br>$H^+$ | B<br>Hydrated $NH_4^+$ |
| Conversion, wt. pct.: | | |
| Naphthalene<br>Alpha $C_{14}$ Olefin<br>Total Alkylated Lube, wt. pct. | 38<br>22<br>26 | 75<br>38<br>47 |
| Product Distribution, wt. pct.: | | |
| Mono-Alkylated<br>Di-Alkylated<br>Dimer | 85<br>8<br>7 | 92<br>8<br>0 |

As shown, the conversion of $H^+$ to hydrated $NH4^+$ form USY zeolite increases significantly catalyst activity. The conversion of naphthalene and olefin increase from 38 and 22 to 75 and 38 wt. percent, respectively. Furthermore, even with an increase in conversion, the hydrated ammonium cationic form of the USY catalyst provides excellent product selectivity toward mono-alkylated products in comparison with Example 2 (from 85 to 92 percent). In addition, the presence of larger hydrated ammonium cation completely suppresses olefin oligomerization as shown by the disappearance of olefin dimers.

Example 4

In this Example, the experiment was carried out under identical conditions as in Example 3 but using a hydrated sodium USY instead of hydrated ammonium USY. The hydrated sodium catalyst contains about 2.4 wt. percent Na (Catalyst C). Table 4 shows that in comparison with the hydrated ammonium catalyst, the hydrated $Na^+$ USY catalyst further improves alkylation activity as shown by an increase in total alkylated yield from 47 to 62 wt. percent with an excellent mono-alkylated product selectivity with little dimer formation. The observed improvement is correlated with the larger ionic dimension of the hydrated $Na^+$ cation as shown in Table 3, which also shows that the larger hydrated ionic radii are associated with cations of smaller radius in the non-hydrated form.

Table 4

| Example No | 1 | 3 | 4 |
|---|---|---|---|
| Cationic Form<br>Ionic Radii, Å<br>Hydrated Radii, Å | $H^+$<br><0.5<br>- | $NH_4^+$<br>1.48<br>3.31 | $Na^+$<br>0.95<br>3.58 |
| Conversion, wt. pct.: | | | |
| Naphthalene<br>Alpha Olefin<br>Alkylated Lube, wt. pct. | 38<br>22<br>26 | 75<br>38<br>47 | 96<br>51<br>62 |
| Product Distribution,wt. pct.: | | | |
| Mono-alkylated<br>Di-Alkylated<br>Dimer | 85<br>8<br>7 | 92<br>8<br>0 | 89<br>10<br>1 |

The following Examples demonstrate that the improvement in aromatic alkylation activity and mono-alkylated product selectivity by modification with larger size cations is also observed with a bound USY catalyst containing a support such as alumina, silica or a clay.

9

Example 5

The alkylation experiment was carried out under identical process conditions as in Example 1. As an example, a commercially available USY catalyst was evaluated in this experiment. The catalyst is an FCC catalyst containing approximately 40 wt% USY component with an unit cell size of 24.55 Å. This particular FCC USY catalyst (Catalyst D) is in hydrated ammonium form containing about 0.20 wt. percent Na and 0.90 wt. percent N in ammonium form. About 5 wt. percent of this FCC USY catalyst was used in this Example. The alkylation performance of this catalyst is tabulated in Table 5.

Example 6

In this Example, the hydrated ammonium USY catalyst (Catalyst D) was converted to $H^+USY$ by calcining at 538°C (1000°F) for 24 hours in air. The resulting catalyst (Catalyst E) contains 0.24 wt. percent Na and has an alpha value of 133 and 24.27 Å unit cell size. Table 5 summarizes the performance of this calcined catalyst evaluated under identical process conditions as in Example 5.

Example 7

The calcined USY catalyst (Catalyst E) obtained from Example 6 was converted to $H_3O+$ form by hydrating the catalyst with water vapor for four days to saturation point. This hydronium cation USY catalyst (Catalyst F) was evaluated under identical conditions as in Example 5. The results are summarized in Table 5.

Example 8

The calcined USY catalyst (Catalyst E) was steamed at 788°C (1450°F) for 10 hours in a 45% steam, 55% air atmosphere at 100 kPa (0 psig) to reduce the the catalyst acidity from 133 to 2 alpha. The performance of the steamed USY (Catalyst G) is shown in Table 5.

Example 9

The hydrated $NH_4^+$ USY catalyst (Catalyst D) was modified to hydrated $Na^+$ USY by back ion exchanging with a 1N NaCl solution using a procedure very similar to that in Example 3. The resulted catalyst contains about 1.4 wt. percent Na (Catalyst H). Table 5 shows the catalyst performance for naphthalene alkylation.

Example 10

The hydrated $NH_4^+$ USY catalyst was converted to hydrated $K^+$ USY by ion back-exchanging with a 1N $KNO_3$ solution using a procedure similar to that in Example 3. The resulted catalyst contains 2.4 wt. percent $K^+$ (Catalyst J). The catalyst performance for naphthalene alkylation is shown in Table 5.

10

## Table 5

### Effect of Hydrate Cation on Naphthalene Alkylation
### Performance of USY Catalyst

| Example No | 5 | 6 | 7 | 8 | 9 | 10 |
|---|---|---|---|---|---|---|
| Catalyst | D | E | F | G | H | J |
| USY Form | Hydrated NH$_4$+ | Calcined H+ | Hydrated H$_3$O+ | Steamed H+ | Hydrated Na+ | Hydrated K+ |
| Ionic Radii, Å | 1.48 | <0.5 | 1.50 | <0.5 | 0.95 | 1.33 |
| Hydrated Radii, Å | 3.31 | – | 2.82 | – | 3.58 | 3.31 |
| Conversion, wt. pct.: | | | | | | |
| Naphthalene | 96 | 79 | 63 | 79 | 96 | 100 |
| Alpha Olefin | 54 | 65 | 41 | 68 | 57 | 59 |
| Total Alkylated Lube, wt. pct. | 64 | 68 | 46 | 71 | 66 | 68 |
| Product Distribution, wt. pct.: | | | | | | |
| Mono-Alkylated | 83 | 46 | 70 | 38 | 82 | 81 |
| Di-Alkylated | 14 | 34 | 24 | 38 | 12 | 12 |
| Tri-Alkylated | – | 7 | – | 15 | – | – |
| Dimer | 3 | 13 | 6 | 9 | 6 | 7 |

The results summarized in Table 5 indicate that all USY zeolites containing hydrated cations of the requisite ionic radii (Catalysts D, H and J) provide nearly complete conversion of naphthalene (>96 wt. percent) and very high selectivity for mono-alkylated naphthalene production (>86 percent). The conversion of hydrated NH$_4$+ (Catalyst D, Example 5) to H+ form (Catalyst E, Example 6) by calcination shifts the product selectivity towards poly-alkylated production. The H+ USY catalyst shows a decrease in naphthalene conversion (79 wt. percent) coupled with an increase in olefin conversion (65 wt. percent). This

11

corresponds to the formation of di- and tri-alkylated naphthalene products, resulting an increase in the viscosity of alkylated naphthalene lube base stocks. The steamed catalyst (Catalyst G) shows further increase in tri-alkylated naphthalene formation. These results indicate that the product selectivity can be effectively controlled by modifying the zeolite catalyst with cations of appropriate ionic size and that the ionic size requirement can usually be satisfied by the use of hydrated cations.

Example 11

The product properties of alkylated naphthalene lube base stocks produced from various USY catalysts are shown in the following Table 6.

Table 6

| Example No | | 5 | 6 | 8 |
|---|---|---|---|---|
| Catalyst | | D | E | G |
| USY Form | | Hydrated $NH_4^+$ | Calcined $H^+$ | Steamed $H^+$ |
| Product Properties: | | | | |
| Pour Point, °C (°F) | | -54 (-65) | -46 (-50) | -40 (-40) |
| KV | @40°C, cSt | 25.30 | 35.54 | 46.30 |
| | @100°C, cSt | 4.32 | 5.68 | 6.97 |
| Viscosity Index | | 60 | 97 | 107 |

Table 6 shows that these alkylated naphthalene synthetic lube base stocks have excellent low-temperature fluidity characteristics as indicated by very low pour point products(<-40°C [40°F]). The lube viscosity index increases along with the lube viscosity as a result of higher poly-alkylated products. These results indicate that by incorporating the bulky cations into the USY catalyst, various viscosity products can be obtained. This illustrates the flexibility of the catalyst system utilizing the cation modification of the zeolite.

Aromatic-containing synthetic lubricants with improved product qualities may be obtained with the use of alkylated naphthalene lube base stocks produced from large-pore zeolite, including USY, in the appropriate cationic form. Modification of the zeolite alkylation catalyst by incorporating bulky cations, preferably in the form of hydrated cations, can effectively control the degree of alkyl substitution and, by so doing, alter the final lube viscosity. In addition, the presence of the cations of the requisite size improves catalyst activity and stability, thus improving overall naphthalene alkylation process economics.

Example 12

This example describes the preparation of zeolite beta catalysts used for the subsequent alkylation experiments. The catalyst is in extrudate form containing 65 wt% zeolite beta, having a silica to alumina ratio of 40, and 35 wt% alumina as a binder. This unsteamed catalyst (Catalyst A) has a high acid activity level as indicated by a 220 alpha measurement. The catalyst acid activity was reduced from 220 to 56 alpha (Catalyst B) and to 36 alpha (Catalyst ) by steaming at 510°C (950°F) for 10 hrs and at 552°C (1025°F) for 16 hrs, respectively. The catalyst properties are set forth in Table 7.

12

Table 7

| Comparison of Zeolite Beta Catalyst Properties | | | |
|---|---|---|---|
| Catalyst No. | A | B | C |
| Alpha | 220 | 56 | 36 |
| Surface Area, m$^2$/g | 454 | 426 | 400 |
| Pore Volume, cc/g | 0.89 | 0.89 | 0.85 |
| Particle Dens., g/cc | 0.79 | 0.80 | 0.81 |

The following examples illustrate the enhancement of zeolite beta alkylation activity by reducing its activity via steaming for the production of high quality alkylated naphthalene lube base stocks.

Example 13

This example demonstrates the catalytic activity of unsteamed zeolite beta catalyst (Catalyst A), prepared according to Ex. 12, for alkylating naphthalene with a long chain alpha olefin. The alkylation reaction was carried out in a 2 gallon autoclave using 2:1 molar ratio of alpha $C_{14}$=:naphthalene, 10 wt% catalyst, at 177°C (350°F) for 9 hrs under a nitrogen pressure of 100 kPa (1 atmosphere). After decanting and filtering the catalyst, the total liquid product was then vacuum distilled at 343°C (650°F) to obtain a 37 wt% alkylated naphthalene lube base stock comprising 88 wt% mono-alkylated naphthalene and 12 wt% di-alkylated naphthalene component. This corresponds to the conversion of 33 wt% naphthalene and 38 wt% olefin. As shown in Table 8, the alkylated aromatic lube produced from the unsteamed zeolite beta catalyst exhibits good product properties including high VI (103 VI) and low pour point (-46°C [-50°F]).

Example 14

In this example, the alkylation reaction was carried out under identical conditions as in Example 13 but using the zeolite beta catalyst steamed to 56 alphas (Catalyst B) of Example 12. As shown in Table 8, this 56 alpha catalyst is more active than the unsteamed 220 alpha catalyst (Catalyst A), providing 68 wt% alkylated lube yield at 71 wt% naphthalene and 67 wt% olefin conversion.

Example 15

This example demonstrates that the alkylation activity is increased further by using the zeolite beta catalyst steamed to 35 alpha. Table 8 indicates that this 36 alpha catalyst at Example 12 provides very high alkylated lube production of 86 wt%.

Table 8

| Effect of Reducing Acidity by Steaming on Alkylation Activity of Zeolite Beta Catalyst | | | | |
|---|---|---|---|---|
| Catalyst No. | | A | B | C |
| Example No. | | 13 | 14 | 15 |
| Catalyst Alpha | | 220 | 56 | 36 |
| Catalyst Performance | | | | |
| Total Alkylated Yield, wt% | | 38 | 68 | 86 |
| Conversion, wt% | | | | |
| Naphthalene | | 33 | 71 | 88 |
| Alpha $C_{14}$ = | | 38 | 67 | 86 |
| Alkylated Lube Properties | | | | |
| Pour Point, °F (°C) | | -50 (-46) | -55 (-48) | -55 (-48) |
| KV | @ 40°C, cSt | 18.55 | 29.80 | 49.54 |
|  | @ 100°C, cSt | 3.899 | 5.205 | 7.261 |
| Viscosity Index | | 103 | 105 | 106 |
| Distribution, wt% | | | | |
| Mono-Alkylated | | 88 | 63 | 41 |
| Di-Alkylated | | 12 | 31 | 42 |
| Tri-Alkylated | | - | 6 | 7 |

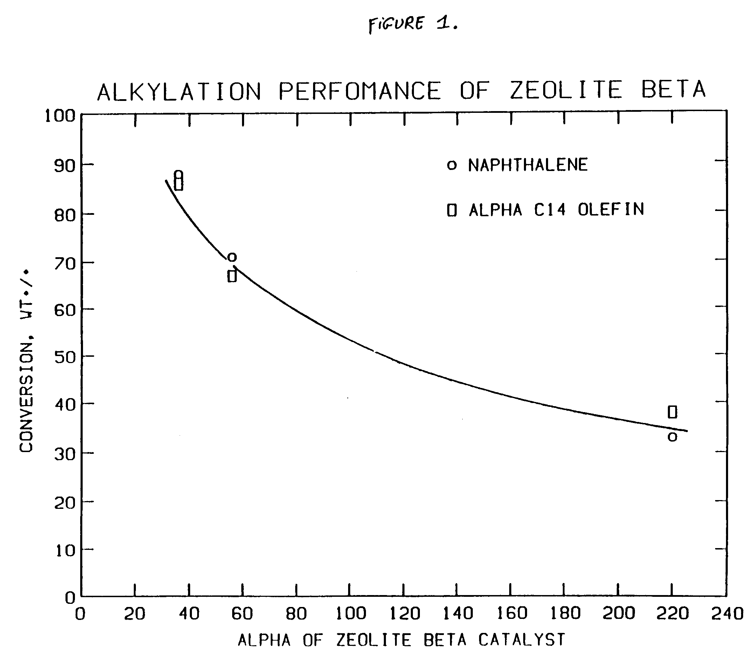

Similar to the results from Table 8, Figure 1 shows that the conversion of naphthalene and alpha $C_{14}$ olefin increase with the decrease in zeolite beta acidity.

Example 16

This example demonstrates that reducing the acid activity of ultrastable zeolite Y (USY) catalyst is also very effective to enhance its alkylation performance. USY catalysts having different acid activity levels (280 alpha for Catalyst D and 55 alpha for Catalyst E) were evacuated for aromatic alkylation. Similar to Example 13, the alkylation experiment was carried out at 204°C (400°F) for 6 hrs using 2.85 wt% catalyst. Table 9 shows the catalyst properties as well as its alkylation performance:

# EP 0 523 165 B1

Table 9

| The Properties And Alkylation Performance Of USY Catalysts | | |
|---|---|---|
| Catalyst No. | D | E |
| Catalyst Properties | | |
| Alpha | 280 | 55 |
| Surface Area, m$^2$/g | 651 | 600 |
| SiO$_2$:Al$_2$O$_3$ Ratio | 5.2 | 14.1 |
| Catalyst Performance | | |
| Total Alkylated Yield, wt% | 39 | 86 |
| Conversion, wt% | | |
| Naphthalene | 54 | 98 |
| α C$_{14}$ Olefin | 34 | 83 |
| Distribution, wt% | | |
| Mono-Alkylated | 82 | 49 |
| Di-Alkylated | 18 | 43 |
| Tri-Alkylated | - | 8 |

The above results indicate the 55 alpha USY catalyst is more active than the 280 alpha USY catalyst, showing increased conversion of naphthalene and aromatic. As a result, the total alkylated lube yield is increased from 39 to 86 wt%.

## Claims

1. A process for preparing long chain alkyl substituted naphthalenes which comprises reacting a naphthalene with an alkylating agent possessing an alkylating aliphatic group having at least six carbon atoms under alkylation reaction conditions and in the presence of an alkylation catalyst comprising a porous crystalline zeolite containing cations having a radius of at least 2.50 Å, to form an alkylated naphthalene possessing at least one alkyl group derived from the alkylating agent.

2. The process of Claim 1 in which the zeolite is a large pore size zeolite having pores with a minimum dimension of at least 7.4 Å.

3. The process of Claim 1 or 2 in which the zeolite has a Constraint Index of not more than 2.

4. The process of Claim 3 in which the zeolite has a Constraint Index of not more than 1.

5. A process of Claim 1 in which the zeolite comprises zeolite X, zeolite Y, or zeolite beta.

6. The process of Claim 5 in which the zeolite comprises zeolite USY.

7. The process of Claim 1 in which the cations have a radius of at least 3.0 Å.

8. The process of Claim 1 in which the cations comprise hydrated cations.

9. The process of Claim 8 in which the hydrated cations comprise hydrated cations of an alkali metal.

10. The process of Claim 9 in which the alkali metal comprises sodium or potassium.

11. The process of claim 8 in which the hydrated cations comprise hydrated ammonium cations.

12. The process of Claim 8 in which the hydrated cations comprise hydrated hydronium cations.

15

13. A process of any one of Claims 1 to 12 in which the alkylating aliphatic group contains at least 8 carbon atoms.

14. The process of Claim 13 in which the alkylating aliphatic group contains at least 12 carbon atoms.

15. The process of Claim 14 in which the alkylating aliphatic group contains from 14 to 20 carbon atoms.

16. The process of any one of Claims 1 to 15 in which the alkylating agent comprises an olefin.

17. The process of any one of Claims 1 to 16 in which the alkylation reaction conditions include a temperature of from 100°C. to 400°C, a pressure of from 20 to 2500 kPa (0.2 to 25 atmospheres), an WHSV of from 0.1 to 10 and an alkylatable aromatic compound to alkylating agent mole ratio of from 0.1:1 to 50:1.

18. A process of Claim 17 in which the alkylation reaction conditions include a temperature of from 100°C to 300°C, a pressure of from 100 to 500 kPa (1 to 5 atmospheres), a WHSV of from 0.5 to 5 and an alkylatable aromatic compound to alkylating agent mole ratio of from 0.5:1 to 5:1.

19. The process of Claim 1 which comprises reacting naphthalene with a olefin containing at least 8 carbon atoms in the presence of an alkylation catalyst comprising a porous crystalline zeolite containing hydrated cations.

20. The process of claim 19 in which the zeolite is a large pore size zeolite having a minimum pore dimension of at least 7.4 Å.

21. A process of Claims 19 or 20 which comprises reacting naphthalene with a olefin containing from 12 to 20 carbon atoms in the presence of an alkylation catalyst comprising a porous crystalline zeolite having a minimum pore dimension of at least 7.4 Å and the crystal structure of zeolite Y and containing hydrated cations.

22. The process of claim 21 in which the hydrated cations comprise hydrated cations of an alkali metal.

23. The process of claim 21 in which the cations comprise hydrated ammonium cations.

24. The process of any one of Claim 21 to 23 in which the cations comprise hydrated cations which have a radius of at least 3.0 Å.

25. The process of any one of Claims 21 to 24 in which the zeolite is zeolite USY.

26. The process according to claim 20 in which the alkylation is carried out under an an inert gas in a batch reactor in which pressure is maintained at a value of at least 446 kPa (50 psig).

27. The process of claim 20 in which the alkylated naphthalene is contacted with an alkali to remove acidic by-products.

28. The process of anyone of claims 1 to 27 wherein the porous crystalline zeolite has an acid activity comparable to an alpha of 0.5 to 60.

29. The process of claim 28 wherein the zeolite has been treated post formation to reduce its acid activity.

**Patentansprüche**

1. Verfahren zur Herstellung von mit langkettigen Alkylgruppen substituierten Naphthalinen dadurch gekennzeichnet, daß man ein Naphthalin mit einer Alkylierungsverbindung, die eine alphatische Alkylierungsgruppe mit mindestens sechs Kohlenstoffatomen enthält, unter Alkylierungsbedingungen und in der Gegenwart eines Alkylierungskatalysators enthaltend einen porösen, kristallinen Zeolith, der Kationen von einem Radius von mindestens, 2,5 Å beinhaltet, umgesetzt, um ein alkyliertes Naphthalin herzustellen, das mit mindestens einer Alkylgruppe der Alkylierungsverbindung substituiert ist.

**2.** Verfahren nach Anspruch 1 dadurch gekennzeichnet, daß als Zeolith ein großporiger Zeolith mit einer minimalen Porendimension von zumindest 7,4 Å eingesetzt wird.

**3.** Verfahren nach Anspruch 1 oder 2 dadurch gekennzeichnet, daß der Zeolith einen Zwangsindex von nicht mehr als 2 aufweist.

**4.** Verfahren nach Anspruch 3 dadurch gekennzeichnet, daß der Zeolith einen Zwangsindex von nicht mehr als 1 aufweist.

**5.** Verfahren nach Anspruch 1 dadurch gekennzeichnet, daß als Zeolith Zeolith X, Zeolith Y oder Zeolith beta eingesetzt wird.

**6.** Verfahren nach Anspruch 5 dadurch gekennzeichnet, daß als Zeolith Zeolith USY eingesetzt wird.

**7.** Verfahren nach Anspruch 1 dadurch gekennzeichnet, daß die Kationen einen Radius von mindestens 3,0 Å haben.

**8.** Verfahren nach Anspruch 1 dadurch gekennzeichnet, daß als Kationen hydratisierte Kationen verwendet werden.

**9.** Verfahren nach Anspruch 8 dadurch gekennzeichnet, daß die hydratisierten Kationen hydratisierte Kationen der Alkalimetalle sind.

**10.** Verfahren nach Anspruch 9 dadurch gekennzeichnet, daß als Alkalimetalle Natrium und Kalium eingesetzt werden.

**11.** Verfahren nach Anspruch 8 dadurch gekennzeichnet, daß als hydratisierte Kationen hydratisierte Ammonium Kationen verwendet werden.

**12.** Verfahren nach Anspruch 8 dadurch gekennzeichnet, daß die hydratisierten Kationen hydratisierte Hydronium Kationen sind.

**13.** Verfahren nach Ansprüchen 1-12 dadurch gekennzeichnet, daß die alkylierende aliphatische Gruppe mindestens 8 Kohlenstoffatome aufweist.

**14.** Verfahren nach Anspruch 13 dadurch gekennzeichnet, daß die alkylierende aliphatische Gruppe mindestens 12 Kohlenstoffatome aufweist.

**15.** Verfahren nach Anspruch 14 dadurch gekennzeichnet, daß die alkylierende aliphatische Gruppe 14-20 Kohlenstoffatome aufweist.

**16.** Verfahren nach Ansprüchen 1-15 dadurch gekennzeichnet, daß als Alkylierungsverbindung ein Olefin verwendet wird.

**17.** Verfahren nach Ansprüchen 1-16 dadurch gekennzeichnet, daß die Alkylierungsreaktion bei Temperaturen von 100°C bis 400°C, Drucken von 20 bis 2500 kPa, gewichtsmäßigen Raumströmungsgeschwindigkeiten von 0,1 bis 10 h$^{-1}$ und Molverhältnissen der zu alkylierenden Aromaten zur Alkylierungsverbindung zwischen 0,1:1 und 50:1 durchgeführt wird.

**18.** Verfahren nach Anspruch 17 dadurch gekennzeichnet, daß die Alkylierungsreaktion bei einer Temperatur von 100°C bis 300°C, ein Druch von 100 bis 500 KPa, einer gewichtsmäßigen Raumströmungsgeschwindigkeit von 0,5 bis 5 h$^{-1}$ und ein Molverhältnis des zu alkylierenden Aromaten zur Alkylierungsverbindung zwischen 0,5:1 und 5:1 durchgeführt wird.

**19.** Verfahren nach Anspruch 1 dadurch gekennzeichnet, daß man Naphthalin mit einem Olefin, das mindestens 8 Kohlenstoffatome hat, in Gegenwart eine Alkylierungskatalysators aus einem porösen, kristallinen Zeolith mit hydratisierten Kationen umsetzt.

EP 0 523 165 B1

**20.** Verfahren nach Anspruch 19 dadurch gekennzeichnet, daß als Zeolith ein großporiger Zeolith mit einer minimalen Porendimension von mindestens 7,4 Å eingesetzt wird.

**21.** Verfahren nach Anspruch 19 oder 20 dadurch gekennzeichnet, daß man Naphthalin mit einem Olefin, das 12-20 Kohlenstoffatome hat, in Gegenwart eines Alkylierungskatalysators aus einem porösen, kristallinen Zeolith mit einer minimalen Porendimension vom mindestens 7,4 Å, einer Kristallstruktur des Zeolith Y und mit hydratisierten Kationen umsetzt.

**22.** Verfahren nach Anspruch 21 dadurch gekennzeichnet, daß als hydratisierte Kationen hydratisierte Kationen der Alkalimetalle eingesetzt werden.

**23.** Verfahren nach Anspruch 21 dadurch gekennzeichnet, daß als Kationen hydratisierte Ammonium Kationen eingesetzt werden.

**24.** Verfahren nach Ansprüchen 21 bis 23 dadurch gekennzeichnet, daß die hydratisierten Kationen einen Radius von mindestens 3,0 Å aufweisen.

**25.** Verfahren nach Ansprüchen 21 bis 24 dadurch gekennzeichnet, daß als Zeolith Zeolith USY verwendet wird.

**26.** Verfahren nach Anspruch 20 dadurch gekennzeichnet, daß die Alkylierung unter einer Inertgasatmosphäre in einem Batch-Reaktor, in dem ein Druck von mindestens 440 kPa gehalten wird, abläuft.

**27.** Verfahren nach Anspruch 20 dadurch gekennzeichnet, daß das alkylierte Naphthalin mit einem Alkali in Kontakt gebracht wird, um die sauren Nebenprodukte zu entfernen.

**28.** Verfahren nach Ansprüchen 1 bis 27 dadurch gekennzeichnet, daß der poröse kristalline Zeolith eine Azidität hat, die einem alpha-Wert von 0,6 bis 60 entspricht.

**29.** Verfahren nach Anspruch 28 dadurch gekennzeichnet, daß das Zeolith nach seiner Herstellung zur Reduzierung seiner Azidität nachbehandelt wurde.

**Revendications**

**1.** Un procédé de préparation de naphtalènes substitués par des radicaux alkyles à chaîne longue, qui comprend la réaction d'un naphtalène avec d'un agent d'alkylation possédant un groupe aliphatique alkylant comportant au moins 6 atomes de carbone dans les conditions réactionnelles d'alkylation et en présence d'un catalyseur d'alkylation comprenant une zéolite cristalline poreuse contenant des cations présentant un rayon d'au moins 2,50 Å, pour former un naphtalène alkylé possédant au moins un groupe alkyle provenant de l'agent d'alkylation.

**2.** Le procédé selon la revendication 1, dans lequel la zéolite est une zéolite à grands pores présentant des pores ayant une dimension minimale d'au moins 7,4 Å.

**3.** Le procédé selon la revendication 1 ou 2, dans lequel la zéolite présente un indice de contrainte qui n'est pas supérieur à 2.

**4.** Le procédé selon la revendication 3, dans lequel la zéolite présente un indice de contrainte qui n'est pas supérieur à 1.

**5.** Le procédé selon la revendication 1, dans lequel la zéolite comprend une zéolite X, une zéolite Y ou une zéolite béta.

**6.** Le procédé selon la revendication 5, dans lequel la zéolite comprend de la zéolite USY.

**7.** Le procédé selon la revendication 1, dans lequel les cations présentent un rayon d'au moins 3,0 Å.

**8.** Le procédé selon la revendication 1, dans lequel les cations comprennent des cations hydratés.

18

**9.** Le procédé selon la revendication 8, dans lequel les cations hydratés comprennent les cations hydratés de métal alcalin.

**10.** Le procédé selon la revendication 9, dans lequel le métal alcalin comprend du sodium ou du potassium.

**11.** Le procédé selon la revendication 8, dans lequel les cations hydratés comprennent des cations ammoniums hydratés.

**12.** Le procédé selon la revendication 8, dans lequel les cations hydratés comprennent les cations hydroniums hydratés.

**13.** Le procédé selon l'une quelconque des revendications 1 à 12, dans lequel le groupe aliphatique alkylant contient au moins 8 atomes de carbone.

**14.** Le procédé selon la revendication 13, dans lequel le groupe aliphatique alkylant contient au moins 12 atomes de carbone.

**15.** Le procédé selon la revendication 14, dans lequel le groupe aliphatique alkylant comprend de 14 à 20 atomes de carbone.

**16.** Le procédé selon l'une quelconque des revendications 1 à 15, dans lequel l'agent d'alkylation comprend une oléfine.

**17.** Le procédé selon l'une quelconque des revendications 1 à 16, dans lequel les conditions de la réaction d'alkylation incluent une température de 100°C à 400°C, une pression de 20 à 2500 kPa (0,2 à 25 atmosphères), une WHSV de 0,1 à 10 et un rapport molaire composé aromatique alkylable/agent d'alkylation de 0,1/1 à 50/1.

**18.** Un procédé selon la revendication 17, dans lequel les conditions de la réaction d'alkylation incluent une température de 100°C à 300°C, une pression de 100 à 500 kPa (1 à 5 atmosphères), une WHSV de 0,5 à 5 et un rapport molaire composé aromatique alkylable/ agent d'alkylation de 0,5/1 à 5/1.

**19.** Le procédé selon la revendication 1 qui comprend la réaction du naphtalène avec une oléfine contenant au moins 8 atomes de carbone en présence d'un catalyseur d'alkylation comprenant une zéolite cristalline poreuse contenant des cations hydratés.

**20.** Le procédé selon la revendication 19, dans lequel la zéolite est une zéolite à grands pores comportant des pores d'une dimension minimale d'au moins 7,4 Å.

**21.** Le procédé selon la revendication 19 ou 20, qui comprend la réaction du naphtalène avec une oléfine contenant de 12 à 20 atomes de carbone en présence d'un catalyseur d'alkylation comprenant une zéolite cristalline poreuse présentant une dimension minimale de pore d'au moins 7,4 Å et la structure cristalline de la zéolite Y et contenant des cations hydratés.

**22.** Le procédé selon la revendication 21, dans lequel les cations hydratés comprennent des cations hydratés d'un métal alcalin.

**23.** Le procédé selon la revendication 21, dans lequel les cations hydratés comprennent les cations d'ammoniums hydratés.

**24.** Le procédé selon l'une quelconque des revendications 21 à 23, dans lequel les cations comprennent les cations hydratés qui présentent un rayon d'au moins 3,0 Å.

**25.** Le procédé selon l'une quelconque des revendications 21 à 24 dans lequel la zéolite est une zéolite USY.

**26.** Le procédé selon la revendication 20 dans lequel l'alkylation est mise en oeuvre sous une atmosphère de gaz inerte dans un réacteur en discontinu dans lequel la pression est maintenue à une valeur d'au moins 446 kPa (50 psig).

**27.** Le procédé selon la revendication 20, dans lequel le naphtalène alkylé est mis en contact avec un composé alcalin pour éliminer les sous-produits acides.

**28.** Le procédé selon l'une quelconque des revendications 1 à 27, dans lequel la zéolite cristalline poreuse présente une activité acide comparable à une valeur alpha de 0,5 à 60.

**29.** Le procédé selon la revendication 28, dans lequel la zéolite a été traitée après sa formation pour abaisser son activité acide.

FIGURE 1.

ALKYLATION PERFOMANCE OF ZEOLITE BETA

o NAPHTHALENE

□ ALPHA C14 OLEFIN

CONVERSION, WT·/·

ALPHA OF ZEOLITE BETA CATALYST

EP 0 523 165 B1